# DEMANDE DE BREVET EUROPEEN

(11) **EP 4 817 622 A1**
(43) Date de publication de la demande: **07.10.2026**
(21) Numéro de dépôt: 26170786.3
(22) Date de dépôt: 03.04.2026
(51) Int. Cl.: A61K 35/52, A61D 19/02, A61K 35/741, A61K 35/742, A61K 35/744, A61K 35/747, C12N 5/076, C12N 5/095

(54) **COMPOSITION DE SEMENCE ANIMALE POUR L'INSEMINATION ARTIFICIELLE COMPRENANT UN PROBIOTIQUE ET UTILISATIONS CORRESPONDANTES**

(30) Priorité: 04.04.2025 FR 2503572
(71) Demandeur: Landata-Cobiporc, 35590 Saint-Gilles (FR)
(72) Inventeur: BRARD, Romain, 35000 Rennes (FR)
(74) Mandataire: Vidon Brevets & Stratégie

(57) **Abrégé**

L'invention concerne un liquide séminal animal destiné à l'insémination artificielle des espèces porcines comme les truies, comprenant de la semence animale et un produit dilueur de conservation de semence dépourvu d'antibiotique, dans lequel a été ajouté au moins une souche probiotique vivante, parmi - une souche appartenant à la famille de bactéries *Enterococcaceae,* et/ou - une souche *Lactobacillus reuteri* et/ou - une souche *Bacillus subtilis.*

## Description

### 1. Domaine technique

La présente invention se rapporte au domaine technique général de l'insémination artificielle animale.

Plus précisément, l'invention concerne une composition de semence animale destinée à l'insémination artificielle animale.

L'invention s'applique ainsi notamment, mais non exclusivement, à l'insémination artificielle des espèces porcines comme les truies.

### 2. État de la technique

Dans le domaine de l'insémination artificielle animale, il est usuel de préparer des doses unitaires de semence animale diluée, qui sont obtenues en diluant, au moyen d'un produit dilueur liquide, de la semence animale qui a été prélevée sur un animal.

Dans le cas de l'industrie de l'élevage porcin par exemple, les dilueurs de semence porcine permettent d'augmenter la durée de conservation et de préserver la qualité des doses de semence de verrats. Les dilueurs de semence porcine sont généralement composés d'une solution tamponnée contenant des nutriments, des électrolytes, des antibiotiques et des substances permettant de protéger les spermatozoïdes contre le stress oxydatif.

L'ajout d'antibiotiques dans le dilueur permet de réduire les risques de croissance bactérienne et de contamination de la semence pendant le stockage, le transport, et l'insémination.

Toutefois, l'utilisation d'antibiotiques dans les dilueurs de semence, notamment de semence porcine, présente un risque potentiel de développement de résistances antibiotiques chez les bactéries. En outre, l'utilisation d'antibiotiques est néfaste/délétère pour les spermatozoïdes présents dans la solution.

Les dosages et les combinaisons d'antibiotiques utilisés dans les dilueurs actuels ne sont pas encadrés par la réglementation. Toutefois, les politiques publiques et les orientations voulues pour les élevages (en particulier le plan national Ecoantibio) visent à réduire, voire supprimer, l'usage des antibiotiques dans les filières de production animale.

Il existe par conséquent un besoin fort d'un dilueur de conservation de semence animale, porcine notamment, qui permette de s'affranchir de l'utilisation d'antibiotiques.

### 3. Exposé de l'invention

L'invention répond à ces besoins en proposant un liquide séminal animal destiné à l'insémination artificielle des espèces porcines comme les truies, comprenant de la semence animale et un produit dilueur de conservation de semence dépourvu d'antibiotique, dans lequel a été ajouté au moins une souche probiotique vivante, parmi - une souche appartenant à la famille de bactéries *Enterococcaceae,* et/ou - une souche *Lactobacillus reuteri* et/ou - une souche *Bacillus subtilis.*

Il est donc proposé d'utiliser des micro-organismes probiotiques, appelés probiotiques vivants, en remplacement des antibiotiques dans un dilueur de semence animale destinée à l'insémination artificielle des espèces porcines comme les truies.

Il est connu que les probiotiques sont des bactéries vivantes qui exercent un effet positif sur la santé notamment.

Par contre, à la connaissance de la Demanderesse, il n'avait jamais été mis en lumière que les probiotiques pouvaient être utilisés dans la pratique de l'insémination artificielle animale.

Les inventeurs ont en effet découvert que des souches probiotiques vivantes ne nuisent à la bonne conservation des spermatozoïdes lorsqu'ils sont incorporés dans des dilueurs de semence animale sans antibiotique.

Également, il a été découvert que ces probiotiques colonisent la dose de semence animale, ce qui présente plusieurs avantages, notamment la diminution du développement des bactéries (autrement dit la diminution du risque de développement de pathogènes), et l'amélioration de l'immunité de l'animal.

Ainsi l'invention repose sur une approche originale ayant consisté à étudier l'effet des probiotiques dans des doses de semence animale pour l'insémination artificielle.

Le liquide séminal testé lors des essais est par exemple constitué par le mélange de 9% à 15% en volume de semence et 85% à 91% en volume de dilueur sans antibiotique dont 0,1% à 0,2% en volume de probiotique.

La composition de l'invention destinée à l'insémination artificielle de truies par exemple est caractérisée par une toxicité réduite ou stress réduit vis-à-vis des spermatozoïdes présents dans le matériel séminal tout en assurant une bonne capacité fertilisante pendant plusieurs jours et en prévenant le développement de bactéries.

Le probiotique dans un exemple non limitatif de mise en œuvre est une souche appartenant à la famille de bactéries *Enterococcaceae.* Il peut s'agir par exemple de la souche bactérienne *Enterococcus faecium* comprenant de façon connue des bactériocines qui ne sont pas des antibiotiques mais qui possèdent des propriétés antibiotiques et qui peuvent donc agir contre d'autres bactéries, c'est-à-dire éliminer ou inhiber la croissance de ces dernières.

La souche probiotique vivante dans un autre exemple non limitatif de mise en œuvre est une souche *Lactobacillus reuteri.*

La souche probiotique vivante dans encore un autre exemple non limitatif de mise en œuvre est une souche *Bacillus subtilis.*

Dans d'autres exemples possibles de mise en œuvre, plutôt qu'une seule souche probiotique, il est ajouté plusieurs souches probiotiques vivantes parmi celles énumérées ci-dessus. Ainsi, une souche appartenant à la famille de bactéries *Enterococcaceae, telle* la souche bactérienne *Enterococcus faecium* peut être combinée avec une souche *Lactobacillus reuteri* seule, ou bien avec une souche *Lactobacillus reuteri* et une souche *Bacillus subtilis.* En variante, les souches *Lactobacillus reuteri* et *Bacillus subtilis* peuvent être combinées.

Selon un aspect particulier de l'invention, le liquide séminal animal comprend de 9% à 15% en volume de semence animale et 85% à 91% en volume de produit dilueur dont 0,1% à 0,2% en volume de probiotique vivant.

La quantité de probiotique ajoutée au produit dilueur sans antibiotique est à des concentrations comprises entre 10⁴ et 10⁸ CFU/ml (CFU : unités formatrices de colonies), et de préférence comprise entre environ 10⁵ et 10⁸ CFU/ml, par exemple 10⁷ CFU/ml.

Ceci correspond à 0,01% et 1% par litre, par exemple 0,1% par litre, de probiotique sur le dilueur final (soit 10⁷ CFU). Les inventeurs ont constaté qu'il n'y a pas de perte d'efficacité sur 48h pour une concentration de 0,1 %.

Selon un aspect particulier de l'invention, le probiotique est une bactérie de la famille de bactéries Enterococcaceae.

Selon un autre aspect particulier de l'invention, le probiotique est constitué d'une souche bactérienne *Enterococcus faecium.*

D'autres probiotiques ont été testés et ont permis d'obtenir des résultats concluants.

Selon un aspect particulier de l'invention, le probiotique est la souche *Lactobacillus reuteri.*

La souche Lactobacillus reuteri est une bactérie lactique de la famille des Lactobacillaceae ou lactobacille. Ce lactobacille est naturellement présent dans le tractus gastro-intestinal, dans la cavité buccale et sur la muqueuse vaginale des humains et d'autres animaux.

Selon un aspect particulier de l'invention, le probiotique est la souche *Bacillus subtilis.* L'invention concerne également l'utilisation du liquide séminal animal tel que décrit précédemment pour l'insémination artificielle animale des espèces porcines comme les truies.

L'invention concerne également un procédé de fabrication d'un liquide séminal tel que décrit précédemment, comprenant - une étape d'ajout dans un produit dilueur de conservation de semence dépourvu d'antibiotique d'une ou plusieurs souches probiotiques vivantes, parmi - une souche appartenant à la famille de bactéries *Enterococcaceae,* et/ou - une souche *Lactobacillus reuteri* et/ou - une souche *Bacillus subtilis,* de sorte à obtenir un premier mélange ;
- une étape d'ajout du premier mélange dans une quantité prédéterminée de semence animale de sorte à obtenir un liquide séminal destiné à l'insémination artificielle des espèces porcines comme les truies, puis
- une étape de conditionnement dudit liquide séminal.

### Liste des figures

D'autres caractéristiques et avantages de l'invention apparaîtront plus clairement à la lecture de la description suivante de modes de réalisation particuliers, donnés à titre de simples exemples illustratifs et non limitatifs, et des dessins annexés, parmi lesquels :
[Fig.1] est une photographie prise au cours de l'essai n°3 par un analyseur de semence porcine d'un échantillon de semence porcine incorporant un probiotique du type *Enterococcus faecium ;*
[Fig.2] montre l'évolution de la mobilité des spermatozoïdes en fonction de la présence du Lactiferm à une concentration de 10⁷ CFU/ml au cours de l'essai n°1 ;
[Fig.3] illustre montre l'évolution de la morphologie des spermatozoïdes en fonction de la présence du Lactiferm à une concentration de 10⁷ CFU/ml au cours de l'essai n°1 ;
[Fig.4] montre l'évolution de la viabilité des spermatozoïdes en fonction de la présence du Lactiferm à une concentration de 10⁷ CFU/ml au cours de l'essai n°1 ;
[Fig.5] montre l'évolution de la qualité de la dose de semence porcine supplémentée en probiotiques entre J1 et J5 au cours de l'essai n°2 ;
[Fig.6] présente les résultats de fertilité en fonction des deux types de doses (essai et témoin) au cours de l'essai n°3 ;
[Fig.7] est un graphique montrant la répartition du nombre d'individus en fonction du rang de portée au cours de l'essai n°3 ;
[Fig.8] illustre le nombre de porcelets nés totaux en fonction du groupe (essai ou témoin) au cours de l'essai n°3 ;
[Fig.9] montre pour chacun des différents traitements la moyenne de la mobilité des spermatozoïdes in vitro pour chaque jour d'analyse au cours de l'essai n°4 ;
[Fig.10] présente les mesures de la mobilité globale des spermatozoïdes en présence de probiotiques entre J1 et J5 au cours de l'essai n°5 ;
[Fig.11] présente les résultats des mesures recueillies sur cinq jours d'analyses au cours de l'essai n°6.

### 4. Description détaillée de l'invention

Un liquide séminal selon la présente invention peut être réalisé en mélangeant les composés suivants : 9% à 15% en volume de semence animale et 85% à 91% en volume de produit dilueur sans antibiotique dont des nutriments, des électrolytes, des substances permettant de protéger les spermatozoïdes contre le stress oxydatif, et 0,1% à 0,2% en volume de probiotique vivant.

A titre d'exemple, la concentration en spermatozoïdes porcins dans la suspension de matériel séminal est comprise entre 200 et 1000 millions de spermatozoïdes par millilitre (spz/ml). Avantageusement, la quantité de probiotique vivant ajoutée au produit dilueur est à des concentrations comprises entre 10⁴ et 10⁸ CFU/ml (CFU : unités formatrices de colonies), et de préférence comprise entre environ 10⁵ et 10⁸ CFU/ml, par exemple 10⁷ CFU/ml.

A titre d'exemple, le produit dilueur est le dilueur dénommé BTS (pour « Beltsville Thawing Solution » en anglais) sans agent antibiotique et dont la composition bien connue de l'homme du métier comprend du glucose (37g), du citrate de sodium (6g), du bicarbonate de sodium (1,25g), de l'EDTA (1,25g), du chlorure de potassium (0,75g) et de l'eau distillée (1000ml). D'autres dilueurs dépourvus d'antibiotiques disponibles sur le marché peuvent être utilisés dans le cadre de l'invention.

Le liquide séminal de l'invention est donc obtenu par la mise en contact directe d'une ou de plusieurs souches probiotiques vivantes dans un dilueur de conservation de semence porcine sans antibiotique.

Des tests in vitro et in vivo ont été menés par les inventeurs avec un liquide séminal respectant les plages de valeurs susmentionnées.

Les résultats de ces tests sont donnés ci-après, en référence aux figures 1 à 11.

Ces différents essais menés par les inventeurs montrent que le recours aux probiotiques dans une semence animale a permis d'obtenir des résultats concluants pour empêcher le développement bactérien sans nuire à la bonne conservation des spermatozoïdes.

Ce constat a notamment été effectué avec la souche bactérienne *Enterococcus faecium.* Il a été constaté par ailleurs que d'autres souches de probiotiques permettent d'obtenir des résultats concluants tels que des Lactobacilles de l'espèce *Lactobacillus Reuteri.*

*Enterococcus faecium* est une bactérie probiotique souvent utilisée pour ses potentielles propriétés bénéfiques pour la santé. Il s'agit d'une bactérie à Gram positif de la famille des Enterococcaceae. Elle est naturellement présente dans l'intestin grêle et le côlon chez les humains et les animaux.

Il a été constaté par les inventeurs au cours d'essais que, dans le dilueur de semence porcine, cette souche se développe et consomme les différents nutriments présents dans la solution. Sa présence n'impacte pas la mobilité, la viabilité et l'intégrité des spermatozoïdes porcins.

4.1 Ceci est confirmé par la figure 1 qui est une photographie prise par un analyseur de semence porcine (dénommé SpermVision (marque déposée)) d'un échantillon de semence porcine incorporant un probiotique du type *Enterococcus faecium* au cours de l'essai n°3 décrit ci-après.

Sur cette figure 1, les trajectoires des spermatozoïdes sont indiquées par des arcs. Les encadrements révèlent des défauts morphologiques des spermatozoïdes, présents initialement. Les points sur la tête des spermatozoïdes indiquent les spermatozoïdes immobiles, probablement morts. Les probiotiques sont observables par des points gris peu contrastés, nombreux, en fond d'image.

Différents tests et essais décrits ci-après avec leur date de réalisation ont permis de démontrer la compatibilité des probiotiques, en alternative aux antibiotiques dans les dilueurs de semence, avec les exigences relatives à la bonne conservation de la semence porcine.

### 4.2 Essai n°1 in vitro en date du 17/04/2023

Il a été étudié au cours de cet essai l'impact du probiotique dénommé Lactiferm WS 200 (marque déposée) sur la semence porcine dans le but de trouver une alternative à la mise en œuvre d'antibiotiques dans les dilueurs de conservation de semence porcine. Ce probiotique est composé de la bactérie *Enterococcus faecium.* Cette bactérie à Gram positif est retrouvée dans le tractus intestinal de l'homme et des animaux.

Cet essai confirme la non-spermicidité du probiotique testé à une concentration de 10⁷ CFU/ml.

### Matériel et méthodes

Une solution de dilueur, dite dilueur témoin, utilisé pour cet essai est une solution de dilueur BTS contenant des antibiotiques.

Une autre solution de dilueur BTS sans antibiotique, dite dilueur essai, est préparée, la quantité de probiotique ajoutée au volume dilueur étant égale à 0,0145g soit une concentration de 1,16. 10⁷ CFU/ml.

### Préparation des semences

De la semence pure provenant de cinq verrats différents est utilisée. La concentration spermatique des différents éjaculats est mesurée à l'aide de l'instrument de comptage de cellules Nucleocounter (marque déposée) qui est une technologie permettant la mesure de cellules (concentration), et également de cellules viables (mesures de viabilité). Les semences sont ensuite diluées avec la solution de dilueur comprenant les probiotiques, pour obtenir une concentration finale de 18,75 millions de spermatozoïdes par ml. Le conditionnement des essais est réalisé en tubes de 5 ml.

### Analyse des semences

L'analyse de la conservation de la semence est faite à l'aide de l'analyseur de semence SpermVision (marque déposée). Les critères observés sont la mobilité et la morphologie des spermatozoïdes. Ces mesures sont réalisées au premier jour soit J1 (17/04/23), à J3 (19/04/23), à J5 (21/04/23) et à J8 (24/04/23). La viabilité a été mesurée à J1, à J3 et à J5 au Nucleocounter (marque déposée).

### Analyses des dilueurs

L'analyse du pH, de la valeur en glucose (réfractométrie), ainsi qu'un dénombrement bactérien ont été réalisées à J1 sur le dilueur témoin et sur la solution de probiotique essai.

### Résultats

### Analyse des dilueurs

| | **REFRACTOMETRIE** | **PH** |
|---|---|---|
| TEMOIN | 4,5 | 6.8 |
| ESSAI | 4,4 | 7 |

Le tableau ci-dessus démontre que les mesures des paramètres physico-chimiques des solutions « témoin » et « essai » ne présentent pas de différences notables lors de la solubilisation des formulations.

### Analyse des semences

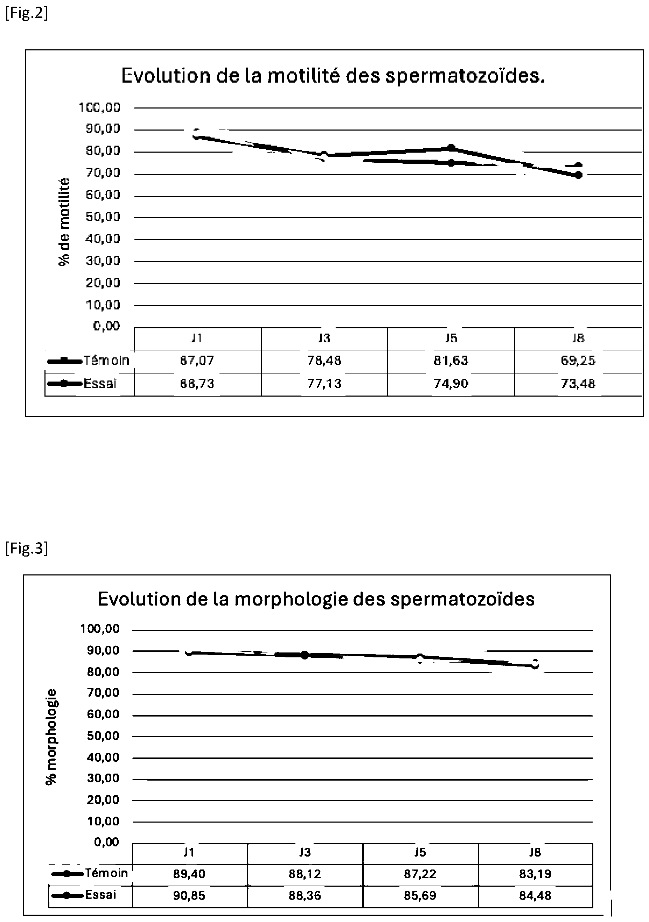

Comme visible sur le graphe de la figure 2 qui montre l'évolution de la mobilité des spermatozoïdes en fonction de la présence du Lactiferm à une concentration de 107 CFU/ml, le pourcentage de spermatozoïdes mobiles a évolué sans présenter de différences entre l'essai et le témoin entre J1 et J3.

A J5, les inventeurs ont observé une diminution de la mobilité des spermatozoïdes, mais n'impactant pas la qualité de semence.

A J8, il est apparu que la qualité de la semence en présence de probiotiques (dilueur essai) était meilleure que celle en présence d'antibiotiques témoin.

Comme visible sur le graphe de la figure 3 qui montre l'évolution de la morphologie des spermatozoïdes en fonction de la présence du Lactiferm à une concentration de 10⁷ CFU/ml, la morphologie « normale » des spermatozoïdes n'a pas été altérée par la présence des probiotiques. Aucune différence n'est à observer entre le dilueur témoin et le dilueur essai de J1 à J8.

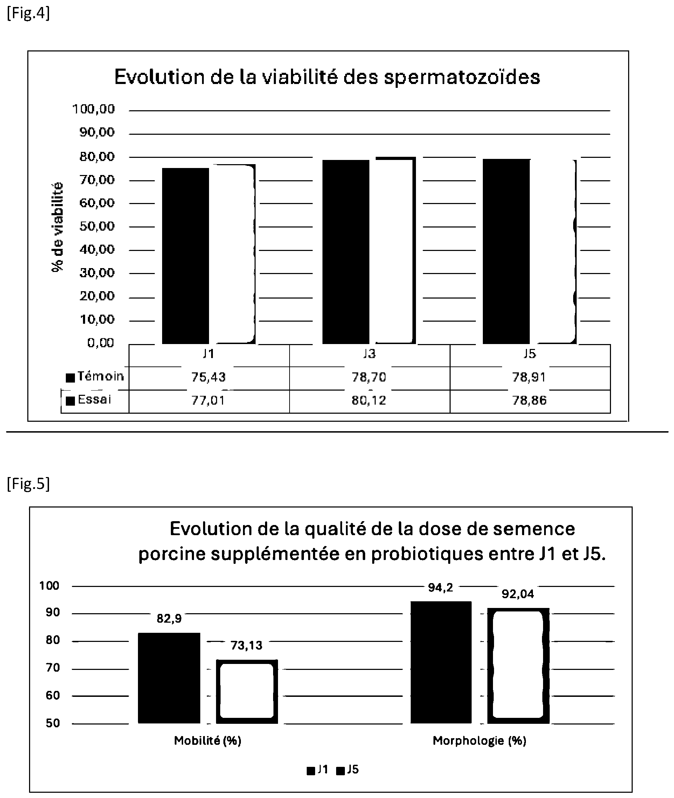

Par ailleurs, comme visible sur le graphe de la figure 4 qui montre l'évolution de la viabilité des spermatozoïdes en fonction de la présence du Lactiferm à une concentration de 10⁷ CFU/ml, la viabilité des spermatozoïdes n'a pas été altérée par la présence des probiotiques. Aucune différence n'est à observer entre le dilueur témoin et le dilueur essai de J1 à J8.

Les résultats obtenus sur cet essai ont donc confirmé la compatibilité in vitro entre la semence porcine et les probiotiques à la concentration de 10⁷ CFU/ml.

Cet essai a démontré l'absence d'impact négatif sur la semence porcine in vitro de la présence de probiotiques dans des doses de semence porcin utilisées pour l'insémination chez un éleveur.

### 4.3 Essai n°2 in vivo en date du 19/07/2023

### Objectif

Après avoir validé *in vitro* la compatibilité des probiotiques et des spermatozoïdes (essai n°1 précédemment décrit), il a été décidé d'observer l'impact in vivo de la présence de probiotiques dans des doses de semence porcine utilisées pour les inséminations artificielles.

Ainsi, des doses de semence porcine supplémentées en probiotiques ont été envoyées en élevage pour un essai in vivo.

### Matériel et méthode

### Élevage

L'élevage essai était constitué de quinze truies qui ont été inséminées, à savoir dix truies dites essai et cinq truies dites témoin. Chaque truie a reçu trois doses de semence porcine supplémentées chacune avec des probiotiques. Les truies ont été sélectionnées par leur âge : au vu de la sensibilité de cet essai, il a été décidé d'utiliser des truies initialement prévues pour la réforme, afin de ne pas trop impacter les résultats de l'éleveur participant à cet essai.

Certaines truies n'ont pas été gardées jusqu'à la mise bas, et ont été réformées pleines, en fonction des choix de l'éleveur.

### Doses de semence supplémentées

Le dilueur utilisé initialement est un BTS sans gentamicine, auquel a été ajouté des probiotiques (Lactiferm 200 WS (marque déposée)) à une concentration de 0,05g/L. Cette concentration correspond à 10⁷ CFU/ml, concentration déterminée comme non-spermicide dans l'essai n°1 précédemment décrit.

Les doses produites chez le Demandeur ont été envoyées le jour même à l'éleveur. Des contrôles physico-chimiques ont été réalisés (pH, réfractométrie). Une dose a été conservée par le Demandeur pour contrôle de la conservation à péremption (J5) par le laboratoire Qualité du Demandeur.

### Résultats

Un échantillon de chaque production a été contrôlé par le laboratoire Qualité du Demandeur.

Les résultats *in vitro* présentés en relation avec la figure 5 montrent l'évolution de la qualité de la dose de semence porcine supplémentée en probiotiques entre J1 et J5.

La conservation de la qualité de semence dans la dose supplémentée en probiotiques est très correcte. Il n'a pas été observé de diminution drastique à J5 de la mobilité et de la morphologie. Ces scores répondent aux critères d'acceptabilité qualité du Demandeur.

Sur les quinze truies inséminées initialement, certaines ont été réformées pendant l'essai. Les effectifs réels de cet essai sont présentés dans le tableau ci-dessous :

| | **Truies jusqu'à l'échographie** | **Truies jusqu'à la mise bas** |
|---|---|---|
| **Témoin** | 5 | 2 |
| **Essai** | 10 | 5 |

Pour ce qui est des résultats des échographies, l'éleveur a relevé un taux de fertilité de 90% pour l'essai et 80% pour le témoin (une truie vide dans chaque groupe). La conclusion principale de ce résultat est que les probiotiques n'ont pas interféré avec la fécondation des ovocytes. Le nombre d'animaux testé restant très faible, aucune conclusion quant à un potentiel impact des probiotiques sur les taux de fertilité n'a pu être émise.

Toutefois, il a été possible de conclure que les truies tolèrent correctement la présence des probiotiques.

Pour ce qui est des mises bas, l'éleveur a relevé un nombre moyen de porcelets nés totaux pour le témoin de 16,5 NT (2 mises-bas).

Pour l'essai, le nombre moyen de porcelets nés totaux est de 21,75 NT (5 mises-bas). Le résultat de prolificité de l'essai s'est donc avéré encourageant. De même que pour la fertilité, une conclusion quant à un impact des probiotiques sur le nombre moyen de nés totaux était précoce. Cependant, il a été possible de conclure que les probiotiques ne perturbent pas, de prime abord, la gestation et la mise-bas des truies.

Cet essai *in vivo* s'est donc avéré positif. Il a été décidé qu'un nombre plus important d'animaux devait être testé, et si possible dans des conditions de conduite d'élevage différentes (conditionnement de la semence porcine, nombre d'insémination par truies, ...).

### 4.4 Essai n°3 in vivo en date du 16/03/2024

L'essai n°2 in vivo consistait en une évaluation préliminaire de l'impact de l'ajout de probiotiques dans la semence porcine diluée lors de l'insémination de truies sur les paramètres de fertilité et de prolificité. Cet essai n°2 a permis de démontrer que le probiotique n'a pas d'impact négatif. En outre, l'absence d'antibiotique n'a pas permis de constater d'infections supplémentaires.

L'objectif de cet essai n°3 in vivo était d'observer l'effet de la souche *Enteroccocus faecium* sur la fertilité et la prolificité des truies inséminées.

Ces éléments ont permis d'obtenir un comparatif avec des doses témoins comprenant un antibiotique (Gentamicine) et un dilueur qui est du BTSA.

Les doses d'essai comprennent un dilueur BTS sans antibiotiques, supplémenté de probiotiques à une concentration de 0,05g/L *d'Enterococcus faecium.* La péremption des doses est définie à J+4 jours. Les doses ont été conservées à 17°C jusqu'à utilisation. La concentration spermatique est d'environ 25 millions spz/mL, dans des doses de 80mL.

Les truies ont été inséminées avec un seul type de dose, à savoir des doses du groupe témoin ou des doses du groupe essai. Les truies du groupe témoin ont reçu en moyenne 2,9 doses, et les truies du groupe essai ont reçu 2,7 doses. Le groupe témoin est constitué de 315 truies, et le groupe essai de 70 truies.

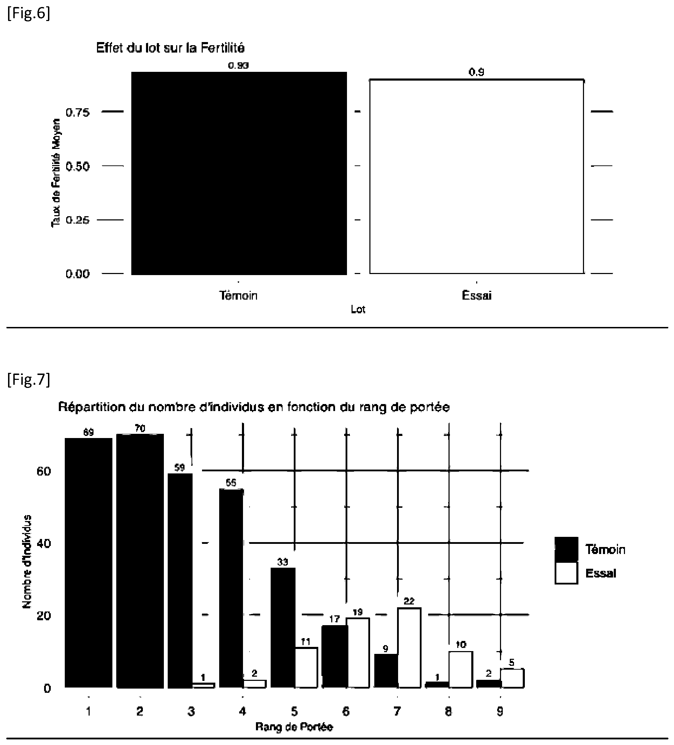

Le graphique de la figure 6 présente les résultats de fertilité (truies pleines à l'échographie) en fonction des deux types de doses (essai et témoin).

Les inventeurs ont observé une lègère diminution de la fertilité avec l'utilisation des doses du groupe essai. Cette différence n'est pas significative (p-value > 0.1).

Les inventeurs se sont interrogés sur l'impact du rang de portée sur la fertilité des truies. En effet, comme convenu avec l'éleveur, ce sont des truies de rang de portée assez élevé qui ont été sélectionnées pour les inséminations avec le groupe essai.

La figure 7 est un graphique montrant la répartition du nombre d'individus en fonction du rang de portée.

Les inventeurs ont observé une répartition inégale des rangs de portée pour les truies des deux groupes. Les truies du groupe témoin sont bien plus jeunes que celles du groupe essai. Cependant, les inventeurs n'ont pas observé de différence significative sur la fertilité pour ces deux groupes (p-value > 0.1) en prenant en compte le facteur Rang de Portée.

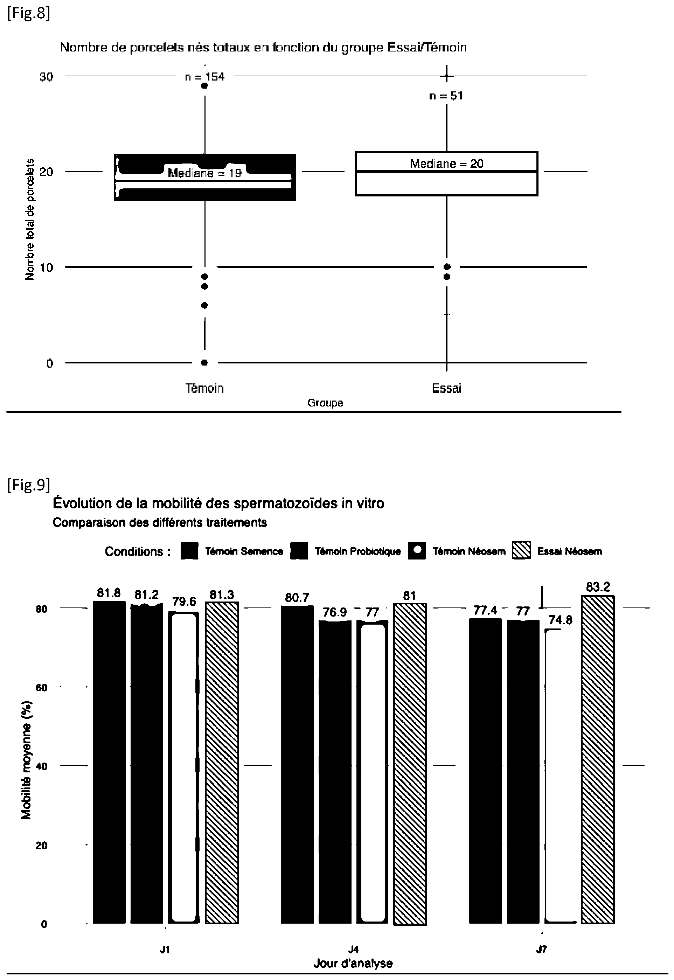

Par la suite, les inventeurs ont comparé l'impact des deux groupes sur la prolificité des truies, c'est-à-dire le nombre de porcelets à la mise-bas. A ce titre, la figure 8 illustre le nombre de porcelets nés totaux en fonction du groupe (essai ou témoin).

Les inventeurs ont observé une légère amélioration du nombre de porcelets nés totaux pour les truies du groupe essai. Cependant, cette différence n'est pas significative entre les deux groupes (p-value > 0.1).

Le facteur Rang de portée a une p-value de 0,07. Cela suggère qu'il y a une tendance pour que le rang de portée affecte le nombre de porcelets nés totaux. Il s'avère que les rangs de portée 4 et 5 présenteraient un nombre plus important de porcelets nés totaux, mais cela n'est pas dû au traitement témoin ou essai.

Ainsi, les inventeurs n'ont pas observé de différence significative sur les deux groupes, que ce soit pour la fertilité ou pour la prolificité. Il a donc été constaté par les inventeurs que le probiotique *Enterococcus faecium* n'a pas eu d'impact sur ces deux indicateurs.

### 4.5 Essai n°4 in vitro en date du 21/01/2025

L'objectif de cet essai n°4 était d'évaluer l'impact des probiotiques sur la mobilité des spermatozoïdes in vitro dans le cadre du conditionnement de la semence porcine dans un conditionnement sous pression dénommé Néosem, différent des blisters de conditionnement.

Les conditions de traitement comprenaient un groupe témoin sans ajout de probiotiques, un groupe témoin avec ajout de probiotiques, un groupe témoin dans des Néosems (un conditionnement sous pression de la semence porcine) sans probiotiques, et un groupe expérimental en Néosem avec des probiotiques.

Le probiotique utilisé ici est un probiotique alimentaire, à savoir la souche *Enterococcus faecium, à* une concentration finale de 0.05g/L dans la semence diluée.

Les mesures de mobilité des spermatozoïdes ont été recueillies à plusieurs jours d'analyse, à savoir les jours 1, 4 et 7, afin d'observer l'évolution de la mobilité en fonction des traitements.

Une analyse statistique a été réalisée pour comparer les différences de mobilité entre les groupes, utilisant le test de Kruskal-Wallis pour tester l'hypothèse de différence des médianes entre les traitements.

Échantillons : 5 verrats.

Conditions : Témoin Semence, Témoin probiotique, Témoin Néosem et Essai Néosem. Concentration : environ 25 millions spz/mL, dans des tubes 5mL ou dans des Néosems de 80ml.

Jours d'analyse : J1/J4/J7.

Mesure : Mobilité des spermatozoïdes (%) mesurée in vitro avec l'analyseur Androvision (marque déposée).

Témoin Semence : Semence diluée dans du BTS dans un tube.

Témoin probiotique : Semence diluée dans du BTS sans antibiotiques et avec ajout de probiotiques dans un tube.

Témoin Néosem : Semence diluée dans du BTS dans un Néosem.

Essai Néosem : Semence diluée dans du BTS sans antibiotiques et avec ajout de probiotiques dans un Néosem.

Le graphique de la figure 9 montre pour chacun des différents traitements la moyenne de la mobilité des spermatozoïdes in vitro pour chaque jour d'analyse.

Les résultats suggèrent que le traitement Essai Néosem présente les meilleures valeurs de mobilité des spermatozoïdes parmi les groupes testés.

En revanche, les traitements témoins montrent une plus grande variabilité, particulièrement dans le groupe "Témoin Probiotique", où une baisse significative de la mobilité a été observée sur certains jours.

Cependant, il est important de noter qu'aucune des conditions n'a montré de différence statistiquement significative après l'analyse par Kruskal-Wallis, indiquant qu'il n'y a pas de preuve concluante que l'un des traitements ait un effet majeur par rapport aux autres.

Cet essai a donc permis de démontrer la compatibilité du probiotique avec autre conditionnement sous pression appelé Néosem différent des blisters.

### 4.6 Essai n°5 in vitro en date du 24 février 2025

Cet essai consistait en un test de spermicidité de deux nouvelles souches probiotiques sur la semence porcine diluée.

Les résultats se sont avérés encourageants, et ont permis d'affirmer que ces deux nouvelles souches de probiotiques testées sont compatibles avec la semence porcine.

### Objectif

Suite aux essais précédents sur la souche *Enterococcus faecium* et sa compatibilité avec la semence porcine diluée, les inventeurs se sont intéressés à deux autres souches bactériennes qualifiées de probiotiques :
- *Lactobacillus reuteri (L. reuteri)* : bactérie Gram⁺ lactique présente dans le microbiote intestinal, y compris chez le porc. Elle produit la reutérine, substance antimicrobienne, et stimule le système immunitaire. Elle est utilisée dans l'élevage porcin en tant que supplément alimentaire. Des bactéries du genre *Lactobacillus* sont naturellement présentes dans le sperme porcin.
- *Bacillus subtilis (B. subtilis)* : bactérie Gram⁺ naturellement présente dans le microbiote. Elle a une action stimulante sur l'immunité de l'hôte, via ses interactions dans le tube digestif. Elle produit des bactériocines qui vont avoir un effet antibactérien contre des pathogènes Gram⁺ *(Clostridium spp, S. aureus,* ...) et Gram⁻ *(E. coli, Salmonella spp, P. aeruginosa,* ...) ³. Tout comme *L. reuteri,* elle est utilisée en supplément alimentaire dans l'élevage porcin.

Ainsi, l'objectif de cet essai *in vitro* était d'évaluer la compatibilité de la semence porcine diluée et des probiotiques *L. reuteri* et *B. subtilis* sur 5 jours.

### Matériel et méthode

Échantillons : 5 verrats
Conditions :
   Témoin : BTS, semence à 25millions/mL de spermatozoïdes, homospermie. Essai A : BTS sans antibiotiques, semence à 25 millions/mL de spermatozoïdes, homospermie et 5.10⁹ CFU/L de *L. reuteri.*
   Essai B : BTS sans antibiotiques, semence à 25 millions/mL de spermatozoïdes, homospermie et 3.10⁷ CFU/L de *B. subtilis.*
Jour d'analyse : cinétique réalisée entre J1 et J5.
Mesure : mobilité des spermatozoïdes à l'Androvision (marque déposée).

Les tubes d'un volume final de 10 mL ont été conservés à 17°C tout au long de l'essai. Les semences ont été réchauffées 30 min à 37°C avant analyse. Des mesures de pH et de réfractométrie ont été réalisées sur les dilueurs avant dilution de la semence porcine.

Les concentrations en CFU/L des bactéries ont été déterminées selon les possibilités de réalisation au laboratoire du Demandeur.

### Résultats

### Paramètres physico-chimiques

Avant dilution de la semence avec les solutions, des mesures de pH et de réfractométrie ont été réalisées et sont détaillées dans le tableau ci-dessous :

| | *pH* | *Réfractométrie* |
|---|---|---|
| *Témoin* | 7.2 | 4.4 |
| *Essai A* | 7.1 | 4.7 |
| *Essai B* | 7.1 | 4.7 |

Il peut ainsi être observé des différences entre les deux essais et le témoin pour la valeur de réfractométrie. Il n'est pas à exclure que les souches bactériennes ajoutées dans les solutions aient un impact sur la quantité de glucose présente, ou bien sur la turbidité globale de la solution (non-visible à l'œil nu).

De plus, pour confirmer la bonne réalisation des essais, des ensemencements sur Uriline (marque déposée) ont été réalisés dans les dilueurs de semence porcine avant utilisation. La lecture a été effectuée 48h après incubation à 30°C. Pour l'essai A (*L. reuteri*), les inventeurs ont observé difficilement des UFC sur les géloses, mais un tapis discret est observé. Pour l'essai B (*B. subtilis*)*,* un tapis entier colonise les géloses.

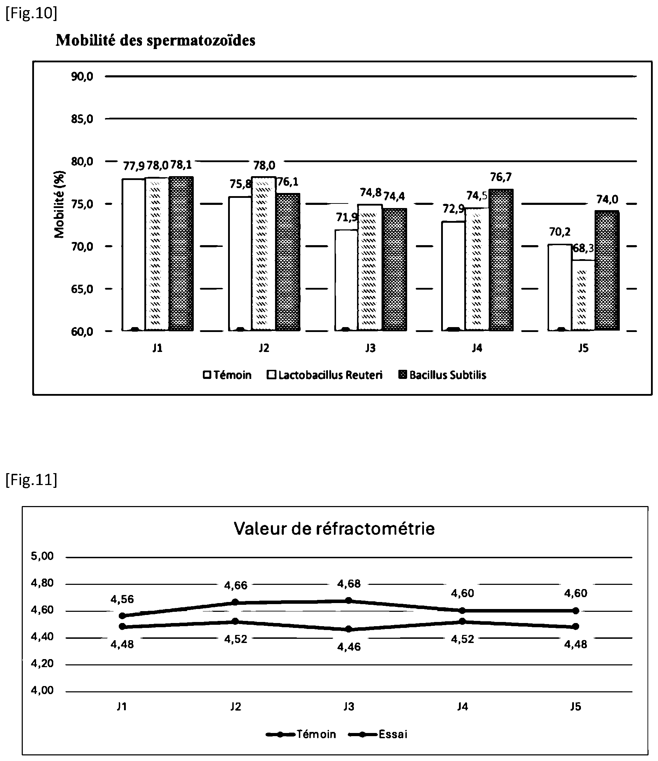

La mobilité globale des spermatozoïdes en présence de probiotiques a été mesurée à l'Androvision (marque déposée), tous les jours de la semaine d'essai entre J1 et J5. Les résultats présentés en figure 10 résultent de la moyenne de mobilité des spermatozoïdes par jour et par verrat.

Ainsi, il a pu être observé que l'ajout de probiotiques dans les essais a été, dans l'ensemble, bénéfique aux spermatozoïdes. Entre J1 et J5, la valeur de mobilité du témoin diminue de 7,7%. Pour l'essai A, *L. reuteri,* la diminution est de 9,7%. Pour l'essai B, *B. subtilis,* la diminution est de 4,2%.

Les probiotiques sont donc compatibles avec les spermatozoïdes porcins. Cependant, nous observons une dégradation plus rapide avec *L. reuteri* en comparaison avec le témoin. Pour ce qui est de *B. subtilis,* il semblerait que la conservation des spermatozoïdes soit améliorée, notamment à J5. Les valeurs de mobilité pour cet essai sont très intéressantes et ouvrent des pistes d'investigations dans les recherches autour de la préservation de la semence porcine.

Afin de vérifier la présence des probiotiques dans les semences après dilution, des Urilines (marque déposée) ont été réalisées à J2 pour chacun des essais. 500µL de semence diluée ont été déposés sur chaque gélose.

Le tableau suivant présente les résultats de dénombrement 48h après l'ensemencement :

| **ECHANTILLON** | **DENOMBREMENT APRES 48H** |
|---|---|
| **A1** | TAPIS |
| **A2** | TAPIS + 3 AUTRES |
| **A3** | TAPIS + 2 AUTRES |
| **A4** | TAPIS + 4 AUTRES |
| **A5** | TAPIS + 15 AUTRES |
| **B1** | TAPIS |
| **B2** | TAPIS |
| **B3** | TAPIS |
| **B4** | TAPIS |
| **B5** | TAPIS |

Tous les ensemencements ont confirmé la présence des probiotiques dans les échantillons. Cependant, avec *L. reuteri,* les inventeurs ont également observé d'autres types bactériens sur les géloses.

### Conclusion et discussion

Ces études préliminaires sur ces nouvelles souches probiotiques se sont avérées encourageantes. Les inventeurs ont pu conclure à la non-spermicidité de ces souches, et à la compatibilité des souches avec le dilueur de conservation de semence porcine.

### 4.7 Essai n°6 en date du 6 janvier 2025

Cette étude in vitro sur les contrôles de réfractométrie a permis aux inventeurs de s'assurer que les bactéries probiotiques présentes dans le dilueur de conservation essai ne consomment pas le sucre disponible, qui est la source d'énergie principale des spermatozoïdes dans notre situation.

### Objectif

Il a été observé l'évolution de la valeur de réfractométrie d'une solution de semence porcine contenant ou non des probiotiques. Le dilueur de conservation de la semence est composé de sucres utilisés par les spermatozoïdes pour leur métabolisme.

Il a été étudié l'utilisation de ces sucres par le probiotique *Enterococcus faecium* lorsqu'il est mis en solution avec les spermatozoïdes. Il apparait que cette souche peut consommer du glucose dans certaines situations. Ces souches ont une forte capacité d'adaptation, et peuvent faire évoluer leur métabolisme en fonction de sources de sucre présentes dans le milieu.

Ainsi, les inventeurs ont voulu mesurer la valeur de réfractométrie des solutions. Cette mesure a permis d'évaluer la quantité de sucre présent dans une solution. Cette technique est principalement utilisée en viticulture, mais est utilisée en contrôle qualité des dilueurs de conservation de semence porcine de la Demanderesse, qui contiennent environ 80% de sucres. Si le probiotique vient à consommer ces sucres, il convient de s'assurer que les spermatozoïdes ne seront pas dépourvu d'une source d'énergie.

### Matériel et méthode

Échantillons : 5 verrats
Conditions: Témoin (dilueur et probiotiques à 0,05g/L) et Essai (dilueur, semence et probiotiques à 0,05 g/L)
Concentration spermatique : 30 millions/ml de semence diluée dans des tubes de 10 mL.
Jour d'analyse : cinétique réalisée entre J1 et J5.
Mesure : valeur de réfractométrie.

Les tubes sont conservés à 17°C tout au long de l'essai. Les mesures sont réalisées sur un volume de 500 µL pour chaque tube analysé.

### Résultats

Le graphique de la figure 11 présente les résultats des mesures recueillies sur cinq jours d'analyses.

Ces résultats suggèrent qu'il n'y a pas de diminution notable entre les deux lots analysés. Sans probiotique, ni spermatozoïdes, la valeur initiale du dilueur de conservation de semence porcine est généralement autour de 4,5. Ici, les valeurs mesurées avoisinent ces résultats.

Pour le témoin, il n'a pas été observé de diminution de la valeur de réfractométrie, qui signifierait une diminution du glucose dans la solution, et donc une potentielle consommation de ce dernier par les probiotiques.

Il en est de même pour l'essai. Il n'y aurait pas de compétition entre les spermatozoïdes et les probiotiques pour le sucre disponible dans la solution.

### Discussion

Ces résultats ont permis de constater que les probiotiques ne consomment pas les sucres présents dans la solution de conservation des spermatozoïdes.

### 4.8 Essai n°7 en date du 14 février 2025

Evaluation de l'impact de l'ajout de probiotiques dans la semence porcine diluée lors de l'insémination de truies sur les paramètres de fertilité et prolificité

### Objectif

Précédemment, au cours de l'essai n°2, les inventeurs ont validé une étude préliminaire in vivo sur quinze truies.

Au cours de l'essai n°3, les inventeurs ont validé une étude préliminaire *in vivo* plus importante en inséminant avec un éleveur partenaire davantage de truies (dont certaines en essai et d'autre en témoin) avec des doses de semence porcine supplémentées en probiotiques. La souche *Enterococcus faecium* avait été utilisée, et les résultats de fertilité et de prolificité étaient satisfaisants dans l'ensemble

Les inventeurs ont poursuivi les inséminations, dans des conditions moins expérimentales que pour le premier essai terrain.

### Matériel et méthode

### Conditions :

Témoin (protocole UB7 : dilueur BTS, concentration spermatique d'environ 25 millions/mL, hétérospermie),
Essai (protocole UB7 modifié : dilueur BTS sans antibiotiques, concentration spermatique d'environ 25 millions/mL, hétérospermie, probiotique à 0,05 g/L).

La péremption des doses est définie à J+4. Elles ont été produites un vendredi, et utilisées entre le samedi et le mardi suivant. Elles sont conservées à 17°C jusqu'à utilisation.
Insémination : 53,3% des animaux ont reçu trois inséminations, 44,4% en ont reçu deux, et 2,2% des animaux n'ont été inséminé qu'une fois. Cette décision revient à l'éleveur, en fonction des observations de chaleur qu'il réalise. Il n'y a pas de croisement dans les doses. L'éleveur s'engage à prévenir le service R&D du Demandeur s'il y a utilisation de produits vétérinaires (antibiotiques ou non).
Animaux : chaque bande de truie est divisée en deux groupes égaux en termes d'effectifs. L'essai est réalisé sur quatre bandes. Au total, le groupe témoin est constitué de 93 truies, et le groupe essai de 87 truies.

### Résultats

### Fertilité des deux groupes de l'essai terrain

Pour rappel, au premier essai terrain, les inventeurs avaient observé une fertilité de 93% pour le témoin, et de 90% pour l'essai. Les résultats recueillis au cours de ce nouveau test présentent une fertilité à 94,3% pour l'essai, et de 100% pour le témoin.

Le test exact de Fisher indique une différence significative entre les groupes témoin et essai (p = 0,0248). Aucun échec n'a été observé dans le groupe témoin, tandis que cinq échecs ont été observés dans le groupe essai. Cela suggère un effet potentiel du traitement sur la fertilité, bien que la forte asymétrie des données empêche une estimation robuste de l'ampleur de cet effet.

### Prolificité des deux groupes de l'essai terrain

Mise-bas de 84 pour témoin (NT=19,76) et 70 pour essai (NT=18,97). Les inventeurs ont observé alors une différence de 0,79 NT en moyenne, en défaveur du groupe essai. Les résultats de cet éleveur sont généralement très élevés, et l'utilisation des probiotiques n'a pas engendré de diminution drastique des résultats pour cet éleveur.

La distribution des données de prolificité ne suit pas une loi normale (test de Shapiro, p-value=0,04). Les inventeurs ont donc réalisé un test non-paramétrique de Mann-Whitney, et la p-value résultante est de 0,318. Ainsi, ils ont pu en conclure que cette différence est non-significative.

### Conclusion et discussion

Le nombre de truies inséminées avec les doses contenant des probiotiques a été augmenté. Ce second essai a permis de confirmer la compatibilité entre les probiotiques et les spermatozoïdes porcins au sein des truies inséminées.

Les différences observées sur les indicateurs de performance de l'élevage, à savoir la fertilité et la prolificité, sont peu importantes, et ces résultats restent très bons. L'élevage participant maintient tout au long de l'année des performances.

Il a été proposé ces doses essais à d'autres élevages, et il a été confirmé que ces résultats ne sont pas simplement influencés par les performances habituelles de l'élevage.

Il a également été testé la souche bactérienne *Enterococcus faecium* combinée avec une souche *Lactobacillus reuteri* seule, et avec une souche *Lactobacillus reuteri* et une souche *Bacillus subtilis.* Il a également été testé la souche *Lactobacillus reuteri* combinée avec la souche *Bacillus subtilis.* Tous ces tests ont également été concluants.

L'ensemble de ces tests visant à ajouter des probiotiques directement dans la semence animale et d'évaluer l'effet antibactérien, a démontré que l'utilisation de probiotiques, seuls ou en combinaison, est une alternative efficace à l'utilisation d'antibiotiques dans la semence animale, telle que la semence porcine.

D'autre part, il est démontré par ces résultats que l'utilisation de probiotiques vient favoriser la conservation des spermatozoïdes.

Il est encore prouvé que l'utilisation de probiotiques empêche le développement bactérien. Les inventeurs ont en effet constaté que le développement de cette souche permet l'inhibition du développement d'autres souches pathogènes retrouvées habituellement dans la semence porcine.

On présente ci-après le mode d'action des différentes souches probiotiques pouvant être mises en œuvre seules ou en combinaison dans un liquide séminal conforme à l'invention. En associant ces différentes souches probiotiques, plusieurs modes d'action sont combinés de sorte à maximiser la couverture protectrice.

### a) Enterococcus faecium :

Produit des bactériocines qui perforent la membrane des bactéries. Très actif contre les Listeria, les Staphylocoques, et quelques entérobactéries.

Compétition écologique : consomme les nutriments du dilueurs, et occupe des sites d'adhésions cellulaires.

Interfère avec la communication bactérienne, ce qui limite les croissances coordonnées de bactéries pathogènes, et la formation de biofilms

Avantage notable : sa présence n'acidifie pas le milieu, ce qui est favorable pour la mobilité des spermatozoïdes.

### b) Lactobacillus reuteri :

Produit de la reutérine : bactériocine très efficace sur l'ADN, les protéines et les membranes des bactéries gram +/- et les levures.

Produit de l'acide lactique, ce qui acidifie le milieu et perturbe le transport membranaire et le métabolisme bactérien. Mais l'effet est limité par le dilueur qui a un pouvoir tampon élevé.

Produit du peroxyde d'hydrogène, très efficace contre les pathogènes, mais peut avoir un effet toxique pour les spermatozoïdes si trop concentré.

Anti-biofilm, car elle empêche l'adhésion bactérienne, et désorganise les biofilms existants. Très efficace contre E.coli, et Pseudo

### c) Bacillus subtilis :

Produit des lipopeptides antimicrobiens (surfactine, iturine et fengycine), qui vont désorganiser les membranes bactériennes, former des pores dans les cellules et engendrer la mort des bactéries. Très actifs contre les bactéries Gram+/- et les champignons.

Produit des enzymes (protéases et nucléases), qui dégradent les biofilms et les débris organiques (ce qui réduit les niches bactériennes).

### Effet anti-biofilms

Compétition nutritive (moins de croissance pour les bactéries pathogènes aérobies notamment).

La ou les souches étant conservées sous format solide, l'ajout du probiotique dans les formulations de dilueur de conservation de semence est réalisable sans imposer de nouvelles contraintes de manipulation du produit.

Les inventeurs se sont également intéressés à l'utilisation d'autres conditionnements, notamment les conditionnements sous pression, et ont validé la compatibilité du substitut avec les différentes formes de conditionnements proposés aux éleveurs.

Bien que plus particulièrement développée pour l'insémination porcine, la solution de l'invention pourra également être utilisée pour tout autre type d'animaux.

## Revendications

1. Liquide séminal animal destiné à l'insémination artificielle des espèces porcines comme les truies, comprenant de la semence animale et un produit dilueur de conservation de semence dépourvu d'antibiotique, dans lequel a été ajouté une ou plusieurs souches probiotiques vivantes, parmi - une souche appartenant à la famille de bactéries *Enterococcaceae,* et/ou - une souche *Lactobacillus reuteri* et/ou - une souche *Bacillus subtilis.*

2. Liquide séminal animal selon la revendication 1, comprenant de 9% à 15% en volume de semence animale et 85% à 91% en volume de produit dilueur dépourvu d'antibiotique dont 0,1% à 0,2% en volume de probiotique.

3. Liquide séminal animal selon la revendication 1 ou 2, la souche de probiotique de la famille de bactéries *Enterococcaceae* étant constitué d'une souche bactérienne *Enterococcus faecium.*

4. Utilisation du liquide séminal animal selon l'une des revendications 1 à 3 pour l'insémination artificielle des espèces porcines comme les truies.

5. Procédé de fabrication d'un liquide séminal selon l'une des revendications 1 à 3 comprenant
- une étape d'ajout dans un produit dilueur de conservation de semence dépourvu d'antibiotique d'une ou plusieurs souches probiotiques vivantes, parmi - une souche appartenant à la famille de bactéries *Enterococcaceae,* et/ou - une souche *Lactobacillus reuteri* et/ou - une souche *Bacillus subtilis,* de sorte à obtenir un premier mélange ;
- une étape d'ajout du premier mélange dans une quantité prédéterminée de semence animale de sorte à obtenir un liquide séminal destiné à l'insémination artificielle des espèces porcines comme les truies, puis
- une étape de conditionnement dudit liquide séminal.
